# EUROPEAN PATENT APPLICATION

(11) **EP 2 985 005 A1**
(43) Date of publication of application: **17.02.2016**
(21) Application number: 14884267.7
(22) Date of filing: 22.08.2014
(51) Int. Cl.: A61D 1/02

(54) **ANIMAL SYRINGE HAVING IMPROVED STRUCTURE**

(30) Priority: 09.04.2014 KR 20140042138
(71) Applicant: Ryu-arm Co., Ltd., Buyeo-gun, Chungcheongnam-do 323-952 (KR)
(72) Inventor: LEE, Jae-Yeop, Buyeo-gun Chungcheongnam-do 323-809 (KR)
(74) Representative: Ter Meer Steinmeister & Partner
(86) International application number: PCT/KR2014/007809
(87) International publication number: WO 2015/156453

(57) **Abstract**

The present invention discloses a medical injector for animals comprising a fixed body comprising a main housing which is made of a housing piece and another housing piece to form a hollow, a working rod placed in the hollow of the main housing and a restoration spring which is in the main housing to offer the restoration power to the working rod; a pressure unit comprising a pressure part which is inserted slidingly to a rear end of the main housing and is coupled integrally to an end of the working rod; and a handle which is formed integrally to the pressure part; a liquid injection unit comprising a liquid cylinder for forming a medical liquid chamber; a which is connected to the working rod to move integrally with the working rod; and a needle unit which is coupled to a front side of the medical liquid cylinder to inject medical liquid of the liquid chamber into the body of animals; and a disinfectant spray unit coupled to the fixed body to spray disinfectant or color liquid to skin of animals.

## Description

### BACKGROUND OF THE INVENTION

### Field of the Invention

Exemplary embodiments of the present invention relates to a medical injector for animals.

### Description of Related Art

The present invention relates to a structure of a medical injector, specially to a medical injector for animals which is capable of continuously easily and quickly injecting animals such as pigs.

Normally, a medical injector is structured only for injecting medical liquid. With medical injectors on the market, injection time for fast moving animals is long, and so it was difficult to inject the exact amount of medical liquid on desired muscle region, and the needle can be easily bended or broken.

Accordingly, a medical injector which is capable of quickly injecting the exact amount on the exact region and Korean Laid-open application 2003-0044400 corresponds to the conventional technology.

However, the conventional art is different from the present invention which uses restoration power of spring for the injection power because in the conventional art the vaccination amount for several times of injection is provided in a medical liquid cylinder at once, and thereafter medical liquid in the cylinder for one time injection is injected by a hitting. That is, in the conventional art, the origin for power to be used for the injection is the hitting power for animal, and the injection needs a hitting power more than a certain magnitude (If the hitting power is small, the injection amount is reduced, or the injection is impossible), and so it is impossible to adjust injection speed or the injection amount per unit time.

In addition, as a conventional art, there are a patent No. 10-0819918 Medical injector for animals and a registration patent No. 10-078476 Medical injector having a marking unit by the applicant. The conventional art has a problem that the operation mechanism and its structure are not compact.

Accordingly, people have needed the development of a medical injector of which a needle is not bended or broken for quick animals such as pigs, and which is capable of injecting exactly an exact amount of medical liquid on a desired muscle region by the uniform injection power, which is mechanically adjusted, regardless of the hitting power of users.

In addition, in the past, it is necessary to have a helper who holds a spray near an injector for making marks one by one with a prominent color in order to distinguish the animals taken injection and hasn't taken injection, and so a large manpower was needed for inoculation, and there have been troublesome problems caused by the marking.

### SUMMARY OF THE INVENTION

An object of the present invention is to provide a medical injector for animals wherein a size of a liquid chamber can be adjusted, and liquid is injected when a user holds a handle and presses tightly, and the medical liquid is charged automatically when the press operation is removed, and wherein the chamber size adjustment is possible and a consumption piston such as o-ring replacement is possible, and a main body can be used semipermanently.

In addition, another object of the present invention is to provide a medical injector for animals having a simple injection operation which has a compact structure without problems that a needle is bended or broken in case of fast moving animals such as pigs

In addition, another object of the present invention is to provide a medical injector for animals having a marking spray unit which is continuously capable of applying the marking liquid (or color disinfectant) on skin of animals by one-hitting and injecting medical liquid into skin.

To obtain the above objects, a medical injector for animals comprises a fixed body 100 comprising a main housing 110 which is made of a housing piece 110a and another housing piece 110b to form a hollow, a working rod 120 placed in the hollow of the main housing 110 and a restoration spring 130 which is in the main housing 110 to offer the restoration power to the working rod 120; a pressure unit 200 comprising a pressure part 210 which is inserted slidingly to a rear end of the main housing 110 and is coupled integrally to an end of the working rod 120; and a handle 220 which is formed integrally to the pressure part 210; a liquid injection unit 300 comprising a liquid cylinder 320 for forming a medical liquid chamber 321; a 330 which is connected to the working rod to move integrally with the working rod 120; and a needle unit 310 which is coupled to a front side of the medical liquid cylinder 320 to inject medical liquid of the liquid chamber 321 into the body of animals; and a disinfectant spray unit 400 coupled to the fixed body 100 to spray disinfectant or color liquid to skin of animals.

### BRIEF DESCRIPTION OF THE DRAWINGS

Fig. 1 is a whole assembly view of a medical injector for animals according to an embodiment of the present invention.
Fig. 2 is a whole sectional view of the medical injector for animals according to the embodiment of the present invention.
Fig.3 is an explosion perspective view of the medical injector for animals according to the embodiment of the present invention.
Fig. 4 is a disassembly view of the medical injector for animals according to the embodiment of the present invention.
Fig. 5 is a disassembly perspective view of a disinfectant spray unit of the medical injector for animals according to the embodiment of the present invention.
Fig. 6 is a plan view of the disinfectant spray unit of the medical injector according to the embodiment of the present invention.
Fig. 7a and 7b are detail views of a medicine chamber size adjustment of the medical injector according to the embodiment of the present invention.
Fig. 8 is a structural view of a conventional spray type marking liquid vessel.
Fig.9 is a structural view of a liquid injection unit according to the embodiment of the present invention.

### DESCRIPTION OF SPECIFIC EMBODIMENTS

Hereinafter, an animal injector of an embodiment of the present invention will be described in detail with respect to accompanying drawings.

As shown in Fibs.1-4, the medical injector of the present invention comprises a fixed body 100, a pressure unit 200, a liquid injection unit 300, and a disinfectant spray unit 400.

The fixed body 100 comprises a main housing 110 which is made of a housing piece 110a and another housing piece 110b to form a hollow, a working rod 120 placed in the hollow of the main housing 110 and a restoration spring 130 which is in the main housing 110 to offer the restoration power to the working rod 120.

As shown in Firs.1-4, the pressure unit 200 comprises a pressure part 210 which is inserted slidingly to a rear end of the main housing 110 and is coupled integrally to an end of the working rod 120; and a handle 220 which is formed integrally to the pressure part 210.

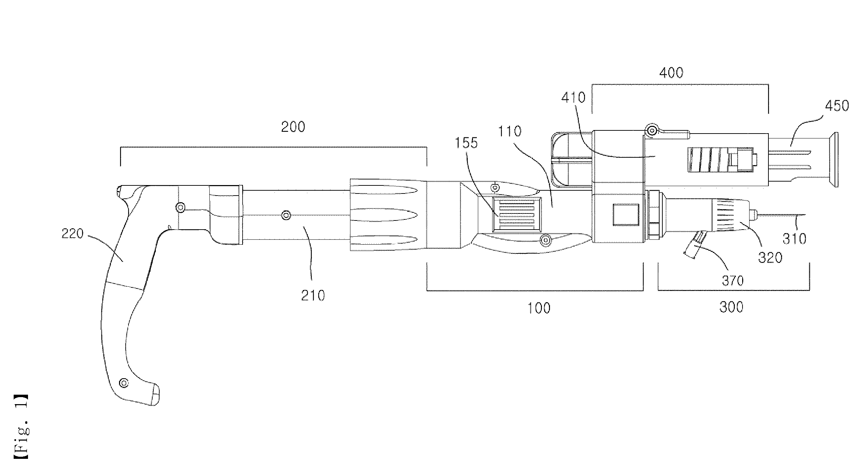

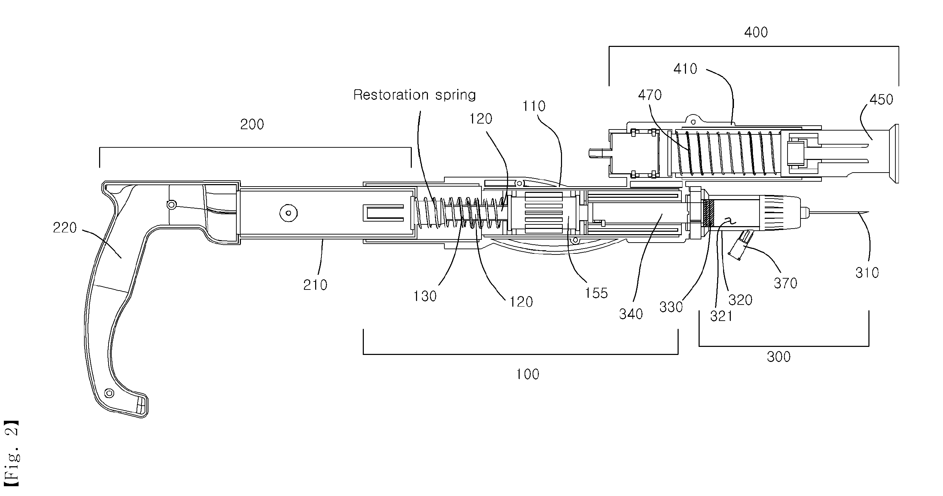

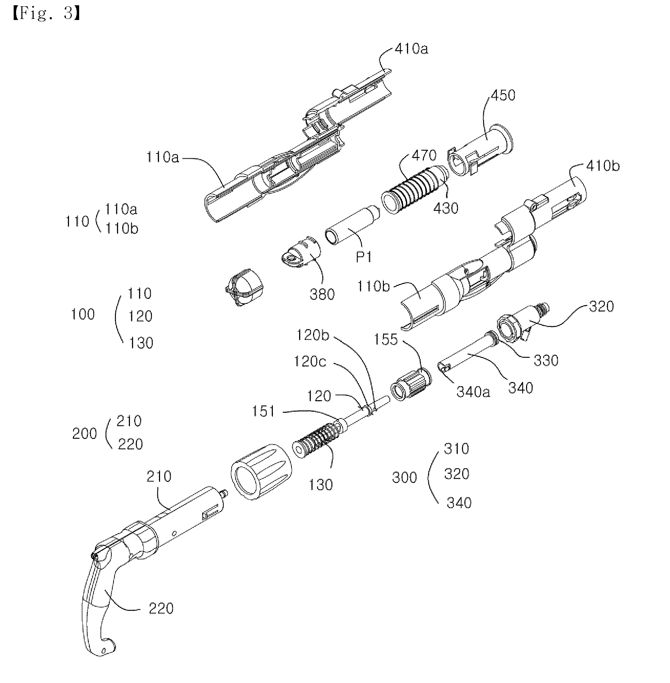

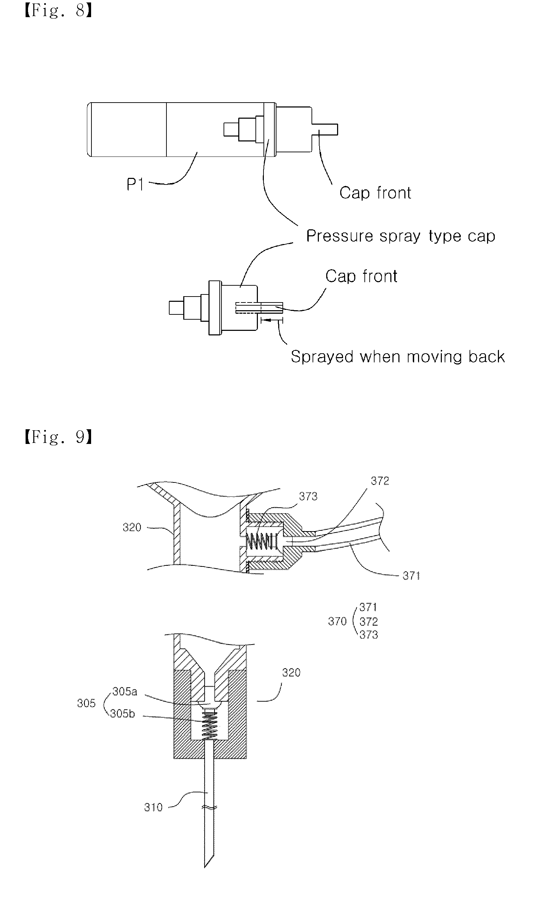

As shown in Figs.1-4 and Fig. 9, the liquid injection unit 300 comprises a liquid cylinder 320 for forming a medical liquid chamber 321; a 330 which is connected to the working rod to move integrally with the working rod 120; and a needle unit 310 which is coupled to a front side of the medical liquid cylinder 320 to inject medical liquid of the liquid chamber 321 into the body of animals.

The disinfectant spray unit 400 is coupled to the fixed body 100 to spray disinfectant or color liquid to skin of animals.

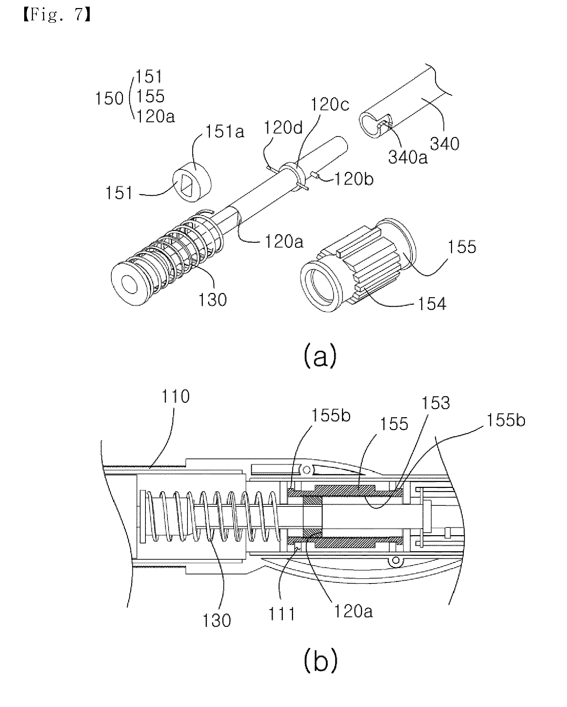

As shown in Figs.1-4 and Fig.7, in the medical injector of the present invention, the fixed body 100 further comprises a chamber size adjustment unit 150 placed in the main housing 110. The chamber size adjustment unit 150 comprises an inner spiral ring 151 having a first spiral 151 a on an outer surface which is inserted slidingly and not-rotatably to the working rod 120; and an outer adjustment ring 155 which is coupled with the spiral ring 151 on an inner surface having the second spiral 153 and has a contact projection 154 on an outer surface, the outer adjustment ring 155 being fixed in the main housing 110.

As shown in Figs.1-4 and Fig.7, in the medical injector for animals of the present invention, a projection step 120a is formed on the working rod 120, and the step is placed on the front of the inner spiral ring 151 inserted in the working rod. If the outer adjustment ring 155 is rotated in the main housing 110 at a fixed position, the inner spiral ring 151 moves forward to push the projection step 120a, and so the working rod 120 and the piston 330 moves forward to reduce the liquid chamber 321, thereby adjusting the size of the liquid chamber 321.

An auxiliary catch unit 155b of the outer adjustment ring 155 is not capable of moving forward and backward because it is caught by a catch groove 111 of the main housing 110, and it is capable of only rotating. An open window 113 is formed on the main housing 110, and the exterior of the outer adjustment ring 155 is exposed through the open window 113.

As shown in Figs.1-4, and Fig. 7, in the medical injector for animals of the present invention, a side of the liquid cylinder 320 is coupled to a front side of the main housing 110 through the auxiliary cap 350 which is detachable by a screw coupling, and a front side of the working rod 120 is coupled to the auxiliary rod 340, and a front side of the auxiliary rod 340 is coupled to the piston 330. A mounting projection 120b is formed in front of the working rod 120. A rear end of the auxiliary rod 340 is a shape of the hollow, and a mounting groove 340a is formed correspondingly to the mounting projection 120b. It is preferable that a corporate body of the auxiliary rod 340 and the piston 330 can be replaced as a new one on its deterioration. It is preferable that both ends of a rotation prevention rod 120d which is mounted in front of the working rod 120 are supported to the main housing 110, and so the working rod 120 is not rotated even though the outer adjustment ring 155 is rotated.

As shown in Figs.1-4, and Fig. 9, in the medical injector for animals of the present invention, a medical liquid charging unit 370 is formed communicately at a side of the liquid cylinder 320, wherein the medical liquid charging unit 370 comprises a supply hose 371 connected to a liquid vessel, a first one way valve 372 for preventing the outflow from the liquid chamber 321 to the supply hose 371, and a valve spring 373 for supporting the one way valve 372.

The liquid injection unit 300 further comprises a counterflow prevention unit 305 between the liquid cylinder 320 and the needle unit 310. The counterflow prevention unit 305 comprises a second one way valve 305a for preventing inflow from animals to the medical liquid chamber 321, and a second valve spring 305b for supporting the second one way valve 3005a.

As shown in Figs.1-3, Fig.5 and Fig.6, in the medical injector for animals of the present invention, the disinfectant spray unit 400 comprises a support housing 410 for forming a second hollow 411 by auxiliary housing pieces 410a, 410b fixed to the main housing 110; a working pipe 431 having the marking liquid vessel P1 which is placed in the auxiliary housing 410 slidingly and is withdrawn by a second distance D2 to press the marking liquid vessel P1 to project the marking liquid in the marking vessel P1; and a front pressure part 450 which is withdrawn by the first distance D1 bigger than the withdrawal distance of the working pipe 430 by the hitting on skin of animals, the front pressure part withdrawing the working pipe 430 by the second distance D2 = (1/20 ~ 1/2) × D1 in the beginning of withdrawal; and a second elastic body 470 of which a front side is connected to the front pressure part 450 and of which a rear side is held up to the auxiliary housing 410 to give restoration power to the front pressure part 450.

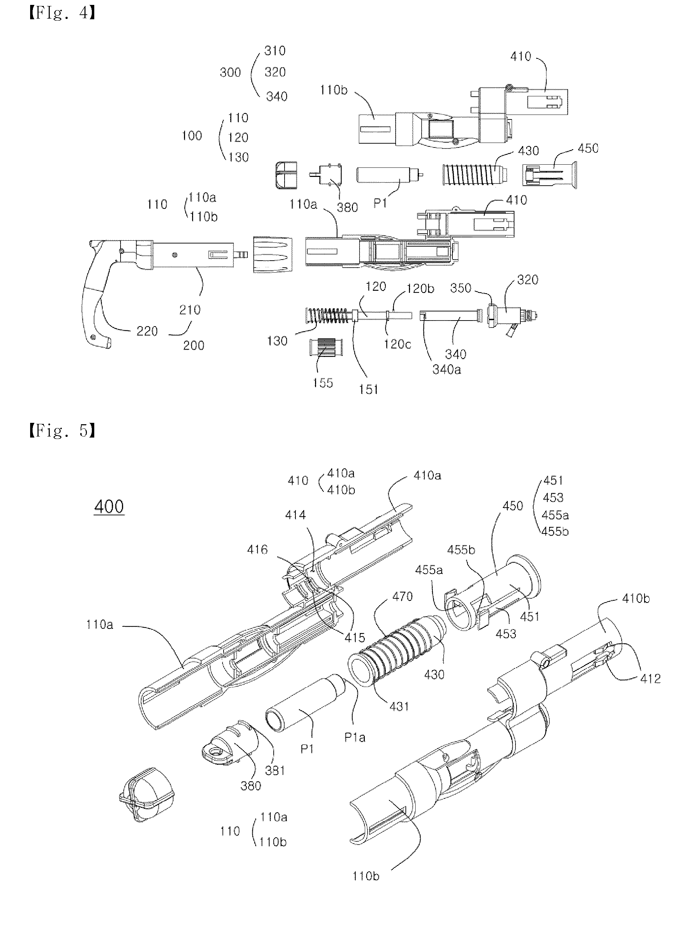

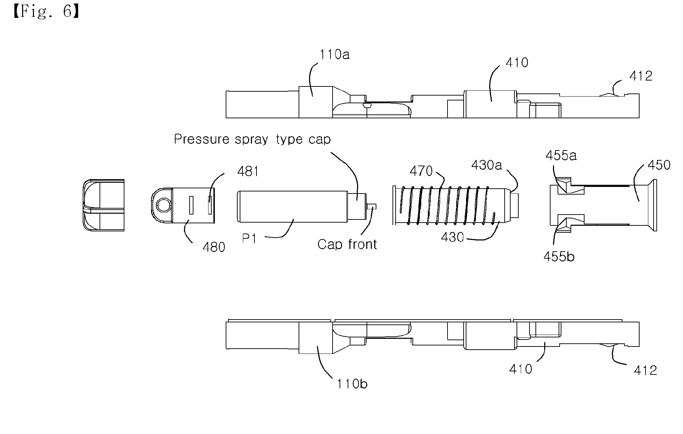

As shown in Fig.5 and Fig.6, in the disinfectant spray unit 400 of the medical injector for animals of the present invention, the front pressure part 450 comprises a hollow pressure pipe 451 into which the working pipe 430 is inserted; a catch wing 453 of which a back side is capable of spreading at both sides of the pressure pipe 451; and first and second sills 455a, 455b which are respectively formed at rear ends of the catch wing 453. The spray unit further comprises a slope 412 for releasing catch which is projected on an outer surface of the auxiliary housing 410, and a rear sill 431 of the working pipe 430 is moved forward and backward in a width of a groove 414 formed in the auxiliary housing 410.

As shown in Fig.5 and Fig.6, in the disinfectant spray unit 400 of the medical injector for animals of the present invention, when the front pressure part 450 is moved back, the first and the second catch sills 455a, 455b are caught at a front surface 430a of the working pipe 430, and the working pipe 430 and the front pressure part 450 are moved back by the second distance D2 smaller than the first distance D1 which is a withdrawal distance of the front pressure part 450, the first and the second catch sills 455a, 455b are moved back along the catch-release slope 412 and spread to the outside thereby releasing the catch at the front surface 430a of the working pipe, and thereafter only the front pressure part 450 is moved back to compress the second elastic body 470.

As shown in Fig. 1-3, Fig.5 and Fig.6, the medical injector for animals of the present invention further comprises an injection frequency counter (not shown) which for sensing one that is selected among the hitting on an animal of the disinfectant spray unit 400, the pressure on the working rod 120 according to a sliding of the pressure part, the medical liquid injection of the liquid injection unit 300 and the liquid charge frequency to display on a display window. A marking liquid vessel P1 is inserted to the working pipe 430, and then is supported by a support cap 480, and by the withdrawl after advance of the support cap 480, an arc-shaped projection 481 formed on an outer circumferential of the support cap 480 is inserted in an arc-shaped groove 416 formed at a back side of the auxiliary housing 410.

By a rotation of a support cap 480, an arc shaped projection 481 formed on an outer circumferential of the support cap 480 escapes from the arc shaped groove 416 formed at a rear side of the auxiliary housing 410, and so the marking liquid vessel P1 is capable of being mounted and changed.

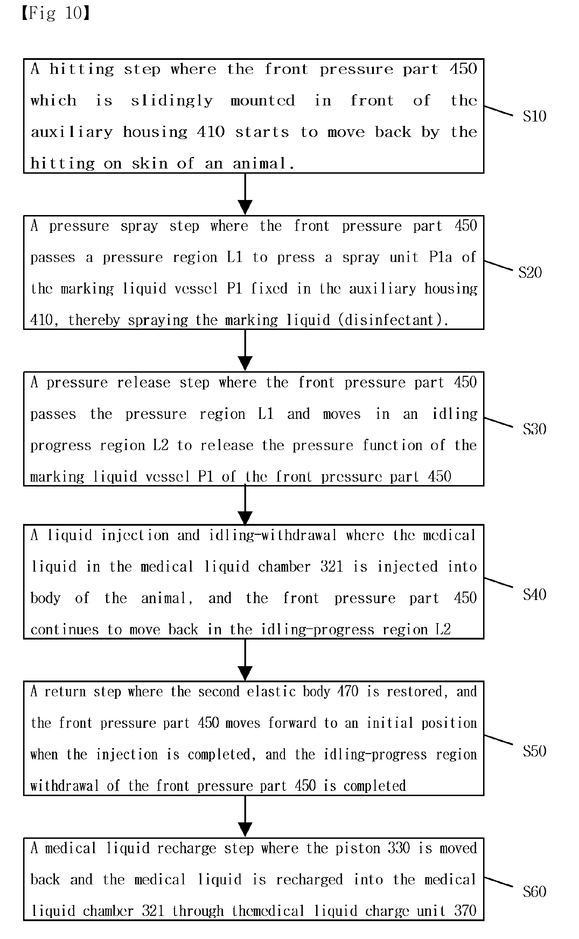

Regarding Fig. 10, an operation of the present invention is described. First, in a hitting step S10, the front pressure part 450, which is slidingly mounted in front of the auxiliary housing 410, starts to move back by the hitting on skin of an animal.

In a pressure spray step S20, the front pressure part 450 passes a pressure region L1 to press a spray unit P1a of the marking liquid vessel P1 fixed in the auxiliary housing 410, thereby spraying the marking liquid (disinfectant).

In a pressure release step S30, the front pressure part 450 passes the pressure region L1 and moves in an idling progress region L2 to release the pressure function of the marking liquid vessel P1 of the front pressure part 450.

In a medical liquid injection and idling-withdrawal S40, the front of the needle unit 310, which is positioned in front of the main housing 110 for supporting the auxiliary housing 410 moves into the skin of an animal, and the piston 330 is pressed to inject medical liquid in the liquid chamber 321 into body of the animal, and the front pressure part 450 continues to move back in the idling-progress region L2.

In a return step S50, when the injection is completed, and the idling-progress region withdrawal of the front pressure part 450 is completed, the second elastic body 470, which has been compressed by the withdrawal of the front pressure part 450, is restored and the front pressure part 450 moves forward to an initial position.

Finally, in a medical liquid recharge step S60, the piston 330 is moved back and the medical liquid is recharged into the medical liquid chamber 321 through the medical liquid charge unit 370

While the present invention has been described with respect to the specific embodiments, it will be apparent to those skilled in the art that various changes and modifications may be made without departing from the spirit and scope of the invention as defined in the following claims.

Reference numbers in following claims is only for helping understanding of the invention, and the reference numbers does not affect interpretation of scope of claims, and claim scope should not be interpreted in a narrow sense by the reference numbers.

## Claims

1. A medical injector for animals comprising:
a fixed body comprising a main housing which is made of a housing piece and another housing piece to form a hollow, a working rod placed in the hollow of the main housing and a restoration spring which is in the main housing to offer the restoration power to the working rod;
a pressure unit comprising a pressure part which is inserted slidingly to a rear end of the main housing and is coupled integrally to an end of the working rod; and a handle which is formed integrally to the pressure part;
a liquid injection unit comprising a liquid cylinder for forming a medical liquid chamber; a which is connected to the working rod to move integrally with the working rod; and a needle unit which is coupled to a front side of the medical liquid cylinder to inject medical liquid of the liquid chamber into the body of animals; and
a disinfectant spray unit coupled to the fixed body to spray disinfectant or color liquid to skin of animals.

2. The medical injector for animals in claim 1, wherein the fixed body further comprises a chamber size adjustment unit placed in the main housing, and the chamber size adjustment unit comprises an inner spiral ring having a first spiral on an outer surface which is inserted slidingly and not-rotatably to the working rod; and an outer adjustment ring which is coupled with the spiral ring on an inner surface having the second spiral and has a contact projection on an outer surface, the outer adjustment ring being fixed in the main housing.

3. The medical injector for animals of claim 2 wherein a projection step is formed on the working rod, and the step is placed on the front of the inner spiral ring inserted in the working rod, and if the outer adjustment ring is rotated in the main housing at a fixed position, the inner spiral ring moves forward to push the projection step, and so the working rod and the piston moves forward to reduce the liquid chamber, thereby adjusting the size of the liquid chamber.

4. The medical injector for animals of claim 1 wherein a side of the liquid cylinder is coupled to a front side of the main housing through the auxiliary cap which is detachable by a screw coupling, and a front side of the working rod is coupled to the auxiliary rod, and a front side of the auxiliary rod is coupled to the piston, and a mounting projection is formed in front of the working rod, and a rear end of the auxiliary rod is a shape of the hollow, and a mounting groove is formed correspondingly to the mounting projection, and a corporate body of the auxiliary rod and the piston can be replaced as a new one on its deterioration.

5. The medical injector for animals of claim 3 wherein both ends of a rotation prevention rod which is mounted in front of the working rod are supported to the main housing, and so the working rod is not rotated even though the outer adjustment ring is rotated.

6. The medical injector for animals of claim 1, wherein a medical liquid charging unit is formed communicately at a side of the liquid cylinder, and the medical liquid charging unit comprises a supply hose connected to a liquid vessel, a first one way valve for preventing the outflow from the liquid chamber to the supply hose, and a valve spring for supporting the one way valve;
and the liquid injection unit further comprises a counterflow prevention unit between the liquid cylinder and the needle unit, and the counterflow prevention unit comprises a second one way valve for preventing inflow from animals to the medical liquid chamber, and a second valve spring for supporting the second one way valve.

7. The medical injector for animals of claim 1 wherein the disinfectant spray unit comprises:
a support housing for forming a second hollow by auxiliary housing pieces, fixed to the main housing;
a working pipe having the marking liquid vessel which is placed in the auxiliary housing slidingly and is withdrawn by a second distance to press the marking liquid vessel to project the marking liquid in the marking vessel; and
a front pressure part which is withdrawn by the first distance bigger than the withdrawal distance of the working pipe by the hitting on skin of animals, the front pressure part withdrawing the working pipe by the second distance D2 = (1/20 ~ 1/2) × D1 in the beginning of withdrawal; and a second elastic body of which a front side is connected to the front pressure part and of which a rear side is held up to the auxiliary housing to give restoration power to the front pressure part.

8. The medical injector for animals of claim 7, wherein the front pressure part comprises a hollow pressure pipe into which the working pipe is inserted; a catch wing of which a back side is capable of spreading at both sides of the pressure pipe; and first and second sills which are respectively formed at rear ends of the catch wing; and
the disinfectant spray unit further comprises a slope for releasing catch which is projected on an outer surface of the auxiliary housing, and a rear sill of the working pipe is moved forward and backward in a width of a groove formed in the auxiliary housing.

9. The medical injector for animals of claim 8 , wherein when the front pressure part is moved back, the first and the second catch sills are caught at a front surface of the working pipe, and the working pipe and the front pressure part are moved back by the second distance smaller than the first distance which is a withdrawl distance of the front pressure part, the first and the second catch sills are moved back along the catch-release slope and spread to the outside thereby releasing the catch at the front surface of the working pipe, and thereafter only the front pressure part is moved back to compress the second elastic body.

10. The medical injector for animals of claim 1, further comprising an injection frequency counter which for sensing one that is selected among a hitting on an animal of the disinfectant spray unit, the pressure on the working rod according to a sliding of the pressure part, the medical liquid injection of the liquid injection unit and the liquid charge frequency, thereby displaying on a display window.
